# DEMANDE DE BREVET EUROPEEN

(11) **EP 3 473 190 A1**
(43) Date de publication de la demande: **24.04.2019**
(21) Numéro de dépôt: 18000813.8
(22) Date de dépôt: 16.10.2018
(51) Int. Cl.: A61B 17/12

(54) **SYSTÈME POUR BOUCHER UN ANÉVRISME**

(30) Priorité: 23.10.2017 FR 1771112
(71) Demandeur: Stsat AG, 1009 Pully (CH)
(72) Inventeur: Plowiecki, Nicolas, 95160 Montmorency (FR); Ramalson, Alain, 95660 Champagen-sur-Oise (FR)
(74) Mandataire: Flavenot, Bernard

(57) **Abrégé**

La présente invention concerne les systèmes pour boucher des anévrismes dont le collet se trouve à la bifurcation de deux artères.

Le système se caractérise essentiellement par le fait qu'il comporte une cuvette 12 apte à être implantée dans l'anévrisme au niveau du collet avec son ouverture 19 tournée vers l'intérieur de l'anévrisme avec un orifice 18 réalisé dans son fond 212 ; une gaine 20 définissant un canal 21 ; des moyens pour relier l'extrémité distale 29 de la gaine 20 à la cuvette 12 de façon que l'orifice de fond 18 et le canal 21 soit en communication directe, et un manchon 30 avec une percée 31, la paroi 32 de ce manchon comportant un point de faiblesse de rupture, le manchon ayant une section transversale hors-tout sensiblement similaire à celle de la gaine 20 et étant en outre monté en coopération en série avec elle de façon qu'ils soient sensiblement en continuité l'un de l'autre, de même que le canal 21 et la percée 31.

## Description

La présente invention concerne les systèmes pour boucher un anévrisme, qui trouvent une application particulièrement avantageuse, notamment, dans le cas d'un anévrisme dont le collet se trouve à la bifurcation de deux artères ou analogues.

Il est connu un traitement de ce type d'anévrisme à l'aide d'un bobinage connu des hommes du métier sous la terminologie "coil" ou "coiling" mais, lorsque le collet de l'anévrisme est large et recouvre une partie de la naissance d'artères en bifurcation, ce traitement ne produit pas correctement l'effet voulu, les coils ayant une forte tendance à s'échapper de l'anévrisme, avec toutes les conséquences qui en découlent.

Pour pallier cet inconvénient, une technique consiste à cathétériser l'une des branches de la bifurcation, sinon les deux, pour y placer des ballons ou des stents qui ont pour but d'empêcher les coils de passer dans les lumières des vaisseaux. Cette technique donne de bons résultats mais la cathétérisation des branches de la bifurcation peut être très difficile, les cathéters ou guides ayant évidemment tendance à aller échouer dans l'anévrisme.

Pour pallier cet inconvénient, il a été mis au point une solution qui consiste en une cuvette qui permet de boucher le collet de l'anévrisme.

Concernant cette technique, peuvent par exemple être cités les documents US2003/171739, EP1006690 et US2007/073334.

Les solutions de l'art antérieur donnent de bons résultats mais présentent malgré tout des inconvénients notamment pour l'implantation de la cuvette et pour le remplissage de l'anévrisme avec des produits comme des coils ou analogues.

La présente invention a pour but de réaliser un système pour boucher un anévrisme qui pallie grande parie les inconvénients mentionnés ci-dessus.

Plus précisément, la présente invention a pour objet un système pour boucher un anévrisme débouchant par un collet sur au moins un vaisseau sanguin, comportant les caractéristiques définies dans la revendication 1 annexée et éventuellement celles définies dans les revendications secondaires.

D'autres caractéristiques et avantages de la présente invention apparaîtront au cours de la description suivante donnée en regard des dessins annexés à titre illustratif, mais nullement limitatif, dans lesquels :
La figure 1 est un schéma de principe d'une vue en coupe longitudinale d'un mode de réalisation du système selon l'invention pour boucher un anévrisme, et
Les figures 2 et 3 représentent deux figures en coupe d'un élément essentiel du système selon l'invention, la figure 2 étant une coupe longitudinale référencée B-B sur la figure 3, et la figure 3 une coupe transversale référencée A-A sur la figure 2.

Il est précisé que, dans la présente description, si l'adverbe "sensiblement" est associé à un qualificatif d'un moyen donné, ce qualificatif doit être compris au sens strict ou approché.

La présente invention est relative à un système pour boucher un anévrisme débouchant par un collet sur au moins un vaisseau sanguin, le système comportant une cuvette 12 apte à être implantée dans l'anévrisme au niveau du collet, avec l'ouverture 19 de la cuvette tournée vers l'intérieur de l'anévrisme.

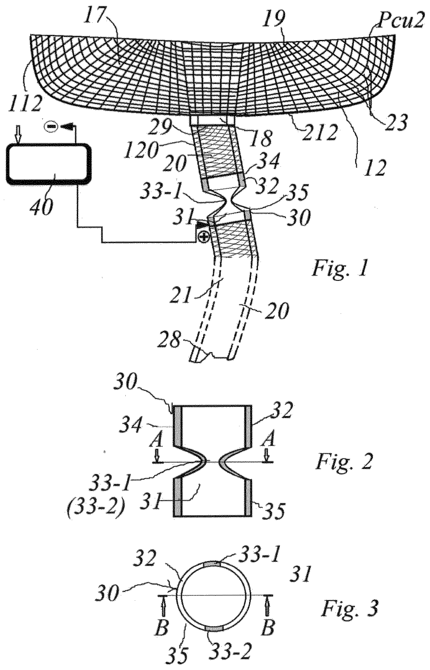

La cuvette comporte, figure 1, un fond 212, un rebord 112 délimitant, avec le fond, un volume intérieur de cuvette 17, et un orifice de fond 18 réalisé dans son fond 212.

Selon une caractéristique de l'invention, le système comporte en outre une gaine 20 définissant, entre son extrémité proximale 28 et son extrémité distale 29, un canal 21 ayant une dimension intérieure au moins égale à la dimension de l'orifice de fond 18 de la cuvette 12, et des moyens pour relier l'extrémité distale 29 de la gaine 20 à la cuvette 12 de façon que l'orifice de fond 18 et le canal 21 soit en communication directe.

Selon une autre caractéristique de l'invention, le système comporte en outre, figures 1 à 3, un manchon 30 définissant une percée traversante axiale 31, la paroi 32 de ce manchon comportant au moins un point de faiblesse de rupture. De façon avantageuse, le manchon a une section transversale hors-tout sensiblement similaire à celle de la gaine 20 et est en outre monté en coopération en série avec cette gaine, figure 1, de façon qu'ils soient sensiblement en continuité l'un de l'autre et que le canal 21 et la percée traversante axiale 31 soient eux aussi sensiblement en continuité l'un de l'autre. Le manchon 30 peut être monté en série sur la gaine 20 par tout moyen dépendant de la nature constitutive de cette gaine. Si, comme il est préférable, cette gaine est constituée d'un tressage de fils métalliques ou analogues, le manchon 30 peut être fixé par collage, soudage, brasure, etc.

Selon une réalisation préférentielle, la cuvette 12 est constituée de façon à être apte à prendre deux états, un premier état dit "replié" instable et un second état dit "déplié" Pcu2 stable (figure 1), la cuvette étant apte à passer, d'elle-même, de son premier état instable à son second état stable Pcu2 quand sont supprimées des forces qui la maintiennent dans son premier état, pour épouser la partie de paroi intérieure de l'anévrisme bordant le collet quand elle est dans son second état Pcu2, et de façon en outre que, quand elle est dans son premier état dit "replié" (non illustré), sa dimension transversale hors-tout soit sensiblement identique à celle hors-tout de la gaine 20.

Selon une réalisation avantageuse, ladite cuvette 20 est réalisée de façon que, lorsqu'elle est dans son second état Pcu2, elle a la forme sensiblement d'une demi-coquille. Elle est ainsi avantageusement constituée de fils à mémoire de forme tressés 23.

De façon très préférentielle, figure 1 à 3, le manchon 30 est constitué de deux manchons secondaires 34, 35 et d'au moins une première patte 33-1 reliant les deux manchons secondaires de façon que ceux-ci soient sensiblement coaxiaux, cette première patte ayant une dimension transversale minimale de très faible valeur par rapport à la dimension transversale de chacun des deux manchons secondaires.

Pour obtenir une bonne stabilisation et une rigidification, le manchon 30 comporte au moins une seconde patte 33-2 reliant les deux manchons secondaires et non confondue avec la première patte 33-1, la somme des dimensions transversales minimales des première et seconde pattes ayant une très faible valeur par rapport à la dimension transversale de chacun des deux manchons secondaires, ces première et seconde pattes 33-1, 33-2 étant avantageusement diamétralement opposées, figure 2 et 3.

Selon une autre caractéristique de l'invention, le système comporte des moyens commandables 40 pour rompre la première patte 33-1, et la au moins seconde patte 33-2 quand elle est présente. De préférence les deux manchons secondaires 31, 32 et la au moins une patte 33-1/-2 sont réalisés d'une seule pièce en un matériau relativement électriquement conducteur qui est choisi pour être apte à être dégradé par au moins l'un des phénomènes suivants : électrolyse, réaction chimique, fusion, une combinaison d'au moins deux de ces phénomènes.

Dans ce cas, le matériau relativement électriquement conducteur est l'un des matériaux suivants : l'acier, le Nitinol, le Titane, optionnellement de préférence le Nitinol.

De préférence, le manchon 30 est monté en série entre les extrémités distale 29 et proximale 28 de la gaine 20, avantageusement à proximité de l'extrémité distale 29, de façon à définir une portion de gaine 120, de relativement faible longueur, entre le manchon 30 et l'orifice de fond 18 de la cuvette 12.

De cette façon, les moyens pour relier la gaine 20 à la cuvette 12 de façon que l'orifice de fond 18 et le canal 21 soient en communication directe sont constitués par le fait que la cuvette 12 et cette portion de gaine 120 sont réalisées d'une seule pièce.

Le système selon l'invention tel que décrit ci-dessus présente une structure qui permet à la cuvette d'être très facilement implantée.

En effet, à cette fin, on utilise un cathéter (non illustré) définissant un conduit dont la section transversale intérieure est au moins égale à la section transversale hors-tout de la gaine 20, de façon que l'ensemble comportant en série : la cuvette 12 dans son premier état dit "replié", la gaine 20 et le manchon 30 puissent facilement glisser en translation dans le cathéter, la gaine et le manchon constituant le poussoir qui permet la mise en place de la cuvette.

Pour implanter la cuvette 12 dans l'anévrisme, l'extrémité distale du cathéter est préalablement positionnée au niveau du collet de l'anévrisme, la cuvette est poussée hors du cathéter et comme elle n'est plus maintenue dans son premier état, elle se déplie dans l'anévrisme en prenant son second état Pcu2, figure 2.

Si, en outre, l'anévrisme doit être rempli avec des produits comme des bobinages connus sous la terminologie "coils", ceux-ci peuvent être introduits dans l'anévrisme via le canal 21, la percée traversante 31 et l'orifice de fond 18 de la cuvette 12, la gaine 20 et le manchon 30 remplissant la fonction de cathéter pour l'introduction de ces coils, ces derniers étant poussés par un autre poussoir dont la section transversale hors tout est au plus égale à celle du canal 21 et de la percée 31, de façon que le poussoir des coils puisse glisser en translation dans la gaine et le manchon.

Quand le collet de l'anévrisme est bouché par la cuvette 12 et que la poche de l'anévrisme est éventuellement remplie de coils dépliés (comme il est bien connu), la rupture des pattes 33-1, 33-2 peut être commandée par les moyens 40 agencés pour, par exemple, réaliser au moyen d'une source d'énergie électrique, une électrolyse sur les parties amincies des pattes.

Dans ce cas, l'anode "+" est par exemple fixée de façon directe ou indirecte sur le manchon 30 et la cathode "-" est par exemple plongée dans le sang du patient (figure 1), la source d'énergie électrique étant activée de façon ad hoc, connue des hommes du métier pour obtenir cette électrolyse.

A ce stade, le cathéter et la partie de gaine comprise entre les restants de la ou des pattes rompues peuvent être retirés du corps du patient, la portion de gaine 120, dans le cas de la réalisation illustrée, et le reste du manchon 30 (c'est-à-dire essentiellement le manchon secondaire 34) pouvant sans inconvénient rester dans le corps du patient.

A la description faite ci-dessus, il est apparent que le système répond aux buts de la présente invention, en palliant en grande partie les inconvénients des systèmes de l'art antérieur.

## Revendications

1. Système pour boucher un anévrisme débouchant par un collet sur au moins un vaisseau sanguin, le système comportant :
• une cuvette (12) apte à être implantée dans l'anévrisme au niveau du collet avec l'ouverture (19) de ladite cuvette tournée vers l'intérieur de l'anévrisme, ladite cuvette comportant un fond (212), un rebord (112) délimitant, avec ledit fond, un volume intérieur de cuvette (17), un orifice (18) réalisé dans son fond (212),
• une gaine (20) définissant, entre son extrémité proximale (28) et son extrémité distale (29), un canal (21), ledit canal ayant une dimension intérieure au moins égale à la dimension dudit orifice de fond (18) de ladite cuvette (12),
• des moyens pour relier l'extrémité distale (29) de ladite gaine (20) à ladite cuvette (12) de façon que ledit orifice de fond (18) et ledit canal (21) soit en communication directe, et
• un manchon (30) définissant une percée traversante axiale (31), la paroi (32) de ce dit manchon comportant au moins un point de faiblesse de rupture, ledit manchon ayant une section transversale hors-tout sensiblement similaire à celle de ladite gaine (20) et étant monté en coopération en série avec ladite gaine de façon qu'ils soient sensiblement en continuité l'un de l'autre, et que ledit canal (21) et ladite percée traversante axiale (31) soient sensiblement en continuité l'un de l'autre, **caractérisé par le fait que** ledit manchon (30) est constitué de deux manchons secondaires (34, 35) et d'au moins une première patte (33-1) et seconde patte (33-2) non confondue avec la première patte (33-1), ces deux dites pattes reliant les deux manchons secondaires de façon que ceux-ci soient sensiblement coaxiaux, ces deux pattes ayant chacune une dimension transversale minimale de très faible valeur par rapport à la dimension transversale de chacun des deux manchons secondaires.

2. Système selon la revendication 1, **caractérisé par le fait que** ladite cuvette (12) est constituée :
• de façon à être apte à prendre deux états, un premier état dit "replié" instable et un second état dit "déplié" (Pcu2) stable, ladite cuvette étant apte à passer, d'elle-même, de son premier état instable à son second état stable (Pcu2) quand sont supprimées des forces qui la maintiennent dans son premier état, pour épouser la partie de paroi intérieure dudit anévrisme bordant ledit collet quand elle est dans son second état (Pcu2), et
• de façon que, quand elle est dans son premier état dit "replié", sa dimension transversale hors-tout soit sensiblement identique à celle de ladite gaine (20).

3. Système selon l'une des revendications 1 et 2, **caractérisé par le fait que**, quand ladite cuvette (20) est dans son second état (Pcu2), elle a la forme sensiblement d'une demi-coquille.

4. Système selon l'une des revendications 1 à 3, **caractérisé par le fait que** les première et seconde pattes (33-1, 33-2) sont diamétralement opposées.

5. Système selon l'une des revendications 1 à 4, **caractérisé par le fait qu'**il comporte des moyens commandables (40) pour rompre les deux pattes (33-1, 33-2).

6. Système selon la revendication 5, **caractérisé par le fait que** les deux manchons secondaires (31, 32) et les deux pattes (33-1, 33-2) sont réalisés d'une seule pièce en un matériau relativement électriquement conducteur.

7. Système selon la revendication 6, **caractérisé par le fait que** ledit matériau relativement électriquement conducteur est choisi pour être apte à être dégradé par l'un des phénomènes suivants : électrolyse, réaction chimique, fusion, une combinaison d'au moins deux de ces phénomènes.

8. Système selon la revendication 7, **caractérisé par le fait que** ledit matériau relativement électriquement conducteur est l'un des matériaux suivants : l'acier, le Nitinol, le Titane, optionnellement le Nitinol.

9. Système selon l'une des revendications précédentes, **caractérisé par le fait que** ledit manchon (30) est monté en série entre les extrémités distale (29) et proximale (28) de ladite gaine (20) de façon à définir une portion de gaine (120) entre le manchon (30) et l'orifice de fond (18) de la cuvette (12), et que les moyens pour relier ladite gaine (20) à ladite cuvette (12) de façon que ledit orifice de fond (18) et ledit canal (21) soient en communication sont constitués **par le fait que** ladite cuvette (12) et ladite portion de gaine (120) sont réalisées d'une seule pièce.
